# EUROPEAN PATENT APPLICATION

(11) **EP 1 988 254 A2**
(43) Date of publication of application: **05.11.2008**
(21) Application number: 06847423.8
(22) Date of filing: 27.12.2006
(51) Int. Cl.: E21B 49/08, G01N 30/02

(54) **CABLE DOWNHOLE GAS CHROMATOGRAPH AND A DOWNHOLE GAS CHROMATOGRAPHY METHOD**

(30) Priority: 29.12.2005 RU 2005141354
(71) Applicant: Services Pétroliers Schlumberger, 75007 Paris (FR); Schlumberger Holdings Limited, Road Town, Tortola (VG); Schlumberger Technology B.V., 2514 JG The Hague (NL); PRAD Research and Development N.V., Willemstad, Curacao (AN)
(72) Inventor: IAKIMOV, Mikhail Nikolaevich, Novosibirskaya obl., 633011 (RU)
(74) Representative: Ilgart, Jean-Christophe
(86) International application number: PCT/RU2006/000704
(87) International publication number: WO 2007/078214

(57) **Abstract**

This invention relates to the devices used in the oil and gas industry. Disclosed herein is the use of a high resolution gas chromatograph in the borehole for borehole fluid type determination in real time mode. The Wireline Downhole Gas Chromatograph comprises a sample chamber with a piston position sensor, the chamber being connected through the sampling valve to the pipeline and through oil pump to hydraulic oil pressure compensation tank; an electric thermostat with a temperature sensor and a chromatograph tube installed inside the thermostat, one side of tube is connected in series through a rotating sample injector, a zeolite filter, a first check valve and a chromatograph isolating valve with a line connecting said sampling valve and said sample chamber, and the other side of tube is connected in series to a second check valve, a fraction detector, a sample portion cylinder and a second pressure gage, wherein said rotating sample injector is connected in series to a pressure reducer, a transporting medium valve, a compressed nitrogen cylinder and a first pressure gage; a bypass line with a bypass valve is connected in parallel to said rotating sample injector, said chromatograph tube and said fraction detector, and the electronic telemetry loop is connected to the output of said fraction detector.

Also disclosed herein is a Downhole gas chromatography method according to which the Downhole gas chromatograph device is lowered into the well to the required depth, and a hydraulic contact with the formation fluids is made; the formation fluids are injected by the pump; the pumping cycle is completed; the sampling valve is opened, and fluid from pipeline is taken into the sample chamber; the sampling valve is closed, and the chromatograph isolating valve is opened to supply the sample for analysis; at the same time the bypass valve is opened in order to wash the contents from previous samples or other unwanted fluids; the transporting medium valve is opened; the rotating sample injector is opened for introducing the fluid sample for analysis; the chromatograph valve is closed, and the sampling valve is opened for fluid supply from the sample fluid chamber into the pipeline; piston is moved by oil pump, the oil pump is stopped, and the sampling valve is closed upon the receipt of a piston position sensor signal; the sample separated in the chromatographic tube is analyzed with the detector; the transporting medium residuals and samples are supplied to the washing section; upon the completion of the analysis, the transporting medium valve is closed. In some embodiments of this invention, said Wireline Downhole Gas Chromatograph is installed in the vicinity of the Modular Dynamic Tester (MDT) bar in a way so that the formation fluid sampling point and the LFA module are at one side, and the pump module (MRPO) or other sampling equipment at the other; during formation fluid injection by the pump, the MDT device performs resistivity, temperature and pressure measurements, and the LFA readings are taken, and the injection cycle is stopped once the required purity is achieved.

## Description

This invention relates to devices used in oil and gas industry.

Gas chromatography is a well-known measurement method based on the introduction of a small amount of the test gas in the permanent flow of a known neutral gas (the carrier gas, or the transporting medium) following which the gases are passed through a capillary pipe. Due to the difference in the mobility of individual gases contained in the sample, the sample is separated in different portions each consisting of an individual gas. At the outlet of the capillary tube these gas portions can be identified using different methods. Thus, the complete chemical composition of the sample can be determined. Advantage of the chromatographic method is the possibility to considerably simplify the design of upstream detectors while retaining the high resolution of the whole system.

Gas chromatography is widely used in oil and gas industry for drilling mud sample analysis and allows to determine the concentrations of H₂S, CH₄, C₂H₆ etc. in gases mixed with the output drilling mud flow. The carrier gas is usually helium or nitrogen.

For the implementation of this method, the equipment used for sampling and formation fluid borehole analysis can be lowered into the well using cable or drilling pipe. The tools are installed on borehole wall, following which a water seal is produced on the wall. Then a sonde is installed in the being tested formation to provide a hydraulic connection with the latter. Formation fluid samples in amounts from several cubic centimeters to hundreds of liters are extracted from the formation by the tool for transferring to the surface and/or in-situ analysis. In some cases the samples are drained back into the well after the completion of in-situ analysis. Fluid analysis methods comprise measurement of fluid specific resistivity, fluid color or light absorption, density (as a function of γ-radiation absorption), gas concentration (by refraction/reflection of gas bubbles in thin pipes) etc. Besides, fluid parameter special measurements are performed: pressure and temperature exact values for different flow modes are recorded using the detectors.

The development of a device allowing borehole analysis methods to be implemented using a gas chromatograph is of special interest. So far, no counterparts of this technical solution have been considered, because chromatographic pipes are very long, and a single analysis session including sample separation into the components of interest requires much time. Moreover, a large amount of gas is taken for a sample. Therefore disadvantage of the existing borehole chromatograph design is its large dimensions and weight making it unsuitable and complex when used in different practical field conditions.

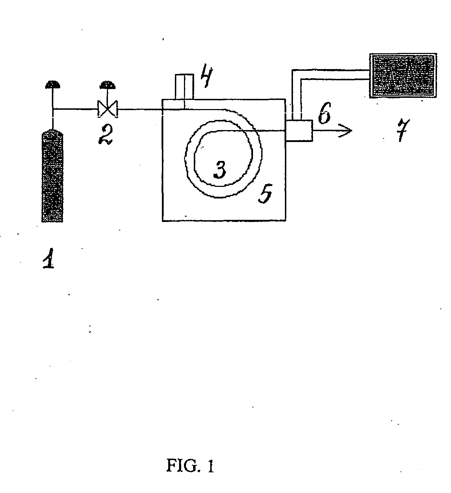

Gas chromatography, especially gas-liquid chromatography, deals with sampies that are preliminarily transferred to the vapor phase and supplied to the head of the chromatographic column (Fig. 1), where: 1 carrier gas, 2 regulating valve, 3 column, 4 sample injection device, 5 thermostat, 6 detector and 7 recorder. The sample is transferred along the column by a flow of mobile inert gas phase. The column itself comprises liquid or solid fixed phase applied to the walls of inert solid material. The transporting carrier gas should be chemically inert. Usually, this is nitrogen, helium, argon or carbon dioxide. The selection of carrier gas often depends on the used detector type. The carrier gas supply system comprises also a molecular sieve for water and other impurities removal.

For optimum chromatograph operation, the sample should not be very large and should be introduced into the column in the form of a 'vapor plug': slow introduction of large samples brings to washing-out the boundaries and to resolution impairment. The most common sample introduction method is those using a microsyringe for sample introduction through a rubber seal into the fast evaporator channel in the column head. The sample channel temperature is usually 50°C above the boiling point of the least volatile sample component. For packed columns, the sample size ranges within tenths of microliter to 20 microliters. For capillary columns, noticeably smaller samples are required, usually about 10⁻³ microliters.

The method of borehole fluid samples has been used since the mid 1960s. Repeat Formation Tester- RFT ™, developed by Schlumberger, Modular Dynamic Tester - MDT (1992) and SRFT™ (1997) are well-known. However, the Repeat Formation Tester could not perform borehole fluid analysis: it was designed for formation fluid samples transference to the surface using two high pressure cylinders equipped with remote controlled valves.

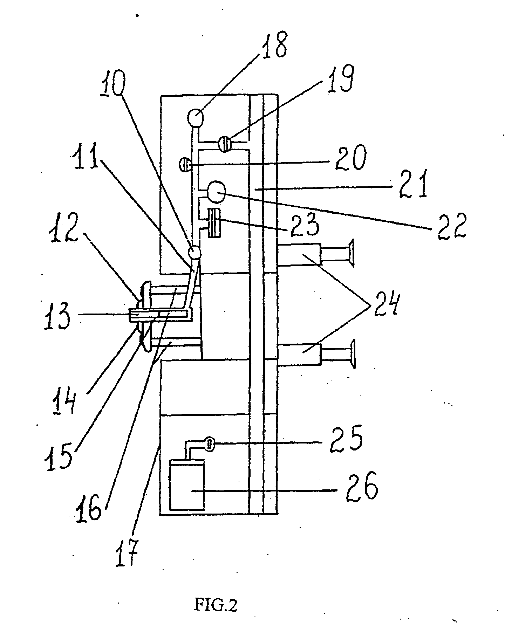

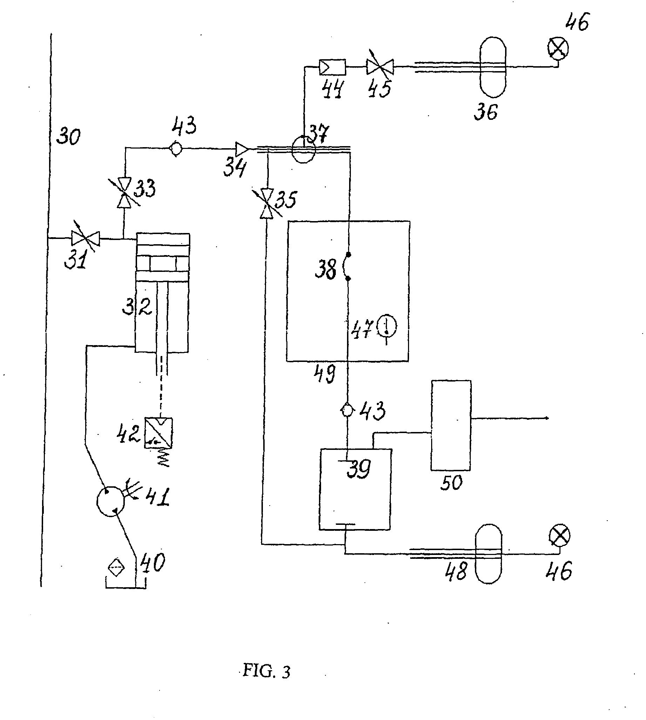

The Modular Dynamic Tester - MDT (Fig. 2) being selected as the prototype was the first device to implement the idea of borehole fluid analysis using the "resistive cell" - a device having four electrodes for measuring the electrical resistance of formation fluids passing through the measuring pipeline (D. Kuchuk, Journal of Petroleum Science and Engineering 11 (1994), p. 123-135). This invention for the first time gave an opportunity to determine the very common type of fluid (e.g. oil or water) before transferring the sample to the surface. Figure 3 shows schematic of the Modular Dynamic Tester - MDT, where 10 is the resistance/temperature cell, 11 is flexible pipe, 12 is the front shoe, 13 is the filter, 14 is the packer, 15 is the filter valve, 16 are the sonde pistons, 17 is the sample module, 18 is the CQG detector, 18 is the isolating valve, 20 is the equalizing valve, 21 is the pipeline, 22 is the preliminary test, 23 is the strain gage, 24 are the rear telescopic pistons, 25 is the throttle/gland valve and 26 is the sample chamber.

Borehole fluid type determination was further developed by optical fluid analyzer (OFA) module introduction. This module is introduced into the pipeline and determines the type of fluids in real time mode transmitting an intense light beam through the fluid and analyzing the spectral parameters of the transmitted light. Due to the different light absorption parameters of oil and water, this recorded spectrum shows the water and oil content and allows determining different types of oil. Gas is analyzed using various detectors that reveal gas bubbles in the reflected light. Common signal record obtained during borehole fluid type determination is shown in Fig. 3. The same measurement principle was used by Schlumberger in the Live Fluid Analyzer (LFA), which is a further development of OFA and is manufactured since 2002, as well as in the Baker-Atlas SampleView module.

However, the complexity of Modular Dynamic Tester design and of its downhole applications didn't allow borehole gas chromatography to be performed using this device.

The object of this invention is to provide a device allowing gas chromatography to be performed for in-situ borehole fluid type determination in real time mode.

The Wireline Downhole Gas Chromatograph disclosed herein is shown in Fig. 3, where: 30 pipeline, 31 sampling valve, 32 sample chamber, 33 chromatograph isolating valve, 34 zeolite filter, 35 bypass valve, 36 liquid nitrogen, 37 the rotating sample injector, 38 chromatograph tube, 39 fraction detector, 40 oil hydraulic pressure compensation tank, 41 oil pump, 42 piston position gage, 43 check valve, 44 pressure reducer, 45 transporting medium valve, 46 pressure gage, 47 temperature sensor, 48 is the sample portion, 49 is the electrical thermostat and 50 is the electronic telemetry loop.

The pipeline is connected to the main pipeline of the MDT modules at the top and the bottom, since:
- The method of introducing the chromatograph into the MDT device by connecting to the common (main) pipeline so that the module can be used both on the top and on the bottom of the sampling point provides flexibility of borehole tool;
- The method and device for gas chromatography performing using a common pipeline allows carrying out multiple analyses in the same borehole section;
- The use of OFA/LFA for chromatographic analysis preparation allows formation fluid analysis to be performed in a way to minimize fluid contamination with drilling mud and drilling mud filtrate.

The Wireline Downhole Gas Chromatograph comprises the appropriate components having the following functions:
1. The sampling valve 31 connects the MDT pipeline 30 with the chromatograph module sample chamber 32;
2. The chromatograph module sample chamber 32 is used for small portions sampling from pipeline 30 for further analysis; the sample chamber is equipped with piston position gage 42 and oil pump 41 allowing samples to be taken from the pipeline or to be placed into the pipeline.
3. The chromatograph isolating valve 33 and check valve 43 allow the sample to be transferred to the detector section of the device and prevent from sample backflow;
4. The changeable zeolite filter 34 ensures sample purity;
5. The bypass line with the bypass valve 35 are used for the removal of unwanted fluids from detector section;
6. The compressed nitrogen cylinder 36 is equipped with the transport medium valve 45 and the pressure reducer 44 that are required for establishing the transporting medium (nitrogen) flow;
7. The rotating sample injector 37 allows fluid sample to be injected in the transporting medium flow;
8. The chromatograph tube 38 in which sample fractions are separated is installed in the electric thermostat 49 that maintains the required increased temperature being regulated with the temperature sensor 47;
9. The fraction detector 39 is based on light absorption;
10. The sample portion cylinder 48 is used for after-test residual fluids gathering; and check valve 43 being installed above the detector prevents from transporting medium backflow;
11. The electronic telemetry loop 50 is used for data transfer to the surface via the wireline recorded data acquisition system.

The device operates according to the following principle:
1. The chromatograph module is located in the vicinity of the MDT bar in a way so that the formation fluid sampling point and the LFA module are at one side, and the pump module (MRPO) or other sampling equipment at the other.
2. The device is lowered into the well to the required depth, and a hydraulic contact with the formation fluids is made.
3. The formation fluids are injected by the pump, at the same time the MDT device performs resistivity, temperature and pressure measurements, and the LFA readings are taken. Once the required purity is achieved, the pumping cycle is stopped, and chromatographic analysis is initiated.
4. The sampling valve in the chromatograph module is opened, and the fluid from the pipeline is taken into the sample chamber.
5. The sampling valve is closed, and the chromatograph isolating valve is opened to supply the sample for analysis.
6. The bypass (mixing) valve is opened instantly in order to wash the contents from previous samples or other unwanted fluids.
7. The transporting medium valve is opened to form a transport medium flow.
8. The rotating sample injector is opened for injection the fluid sample for analysis.
9. The chromatograph valve is closed, and the sampling valve is opened for fluid supply from the sample fluid chamber to the pipeline. The oil pump is used for piston movement. The oil pump is stopped, and the sampling valve is closed upon the receipt of a piston position gage signal.
10. The sample is then separated in the chromatograph tube and analyzed with the detector using the standard gas chromatography method. The transporting medium residuals and the samples are supplied to the washing section.
11. Upon the completion of the analysis, the transporting medium valve is closed, and the device is ready for repeating the test at another well depth beginning from step 2 above.

The ultra small chromatographic pipes designed by the Catalysis Institute specialists of the Siberian Branch of Russian Academy of Sciences gave an opportunity to develop a device allowing to combine the gas chromatography method with oil and gas industry requirements.

The major advantage of the device claimed herein is the considerable reduction of sample processing time due to the small sizes of tubes. Analysis using the device claimed herein can be performed in a few minutes instead of several hours. This allowed the borehole gas analyzer to be developed based on the chromatography principle.

Thus, for the first time gas chromatography is used for borehole fluid type determination in the well in real time mode.

## Claims

1. The use of high resolution gas chromatograph in the borehole for borehole fluid type determination in real time mode.

2. A Wireline Downhole Gas Chromatograph comprising a sample chamber with a piston position detector connected through the sampling valve to the pipeline and through oil pump to a hydraulic oil pressure compensation tank; an electric thermostat with a temperature sensor and a chromatograph tube installed inside the thermostat, one side of tube is connected in series through a rotating sample injector; a zeolite filter; a first check valve and a chromatograph isolating valve with a line connecting said sampling valve and said sample chamber, and the other side of which is connected in series to a second check valve, a fraction detector, a sample portion cylinder and a second pressure gage, wherein said rotating sample injector is connected in series to a pressure reducer, a transporting medium valve, a compressed nitrogen cylinder and a first pressure gage; a bypass line with bypass valve is connected in parallel to said rotating sample injector, said chromatograph tube and said fraction detector; and the electronic telemetry loop is connected to the output of said fraction detector.

3. A Downhole gas chromatography method wherein
- the Downhole gas chromatograph device is lowered into the well to the required depth, and a hydraulic contact with the formation fluids is made;
- the formation fluids are injected by the pump;
- the injection cycle is completed;
- the sampling valve is opened, and the fluid from the pipeline is taken into the sample chamber;
- the sampling valve is closed, and the chromatograph isolating valve is opened to supply the sample for analysis;
- the bypass valve is opened simultaneously in order wash the contents from previous samples or other unwanted fluids;
- the transporting medium valve is opened;
- the rotating sample injector is opened for fluid sample injection for analysis;
- the chromatograph valve is closed, and the sampling valve is opened for fluid supply from the sample fluid chamber to the pipeline;
- piston is moved using oil pump , the oil pump is stopped, and the sampling valve is closed upon the receipt of a piston position sensor signal;
- the sample having been separated in the chromatographic tube is analyzed with the detector;
- the transporting medium residuals and the samples are supplied to the washing section;
- upon the completion of the analysis, the transporting medium valve is closed.

4. The method of claim 3 **characterized** wherein Wireline Downhole Gas Chromatograph is installed in the vicinity of the Modular Dynamic Tester (MDT) bar in a way so that the formation fluid sampling point and the LFA module are at one side, and the pump module (MRPO) or other sampling equipment at the other; during formation fluid injection by the pump, the MDT device performs resistivity, temperature and pressure measurements, and the LFA readings are taken; and the pumping cycle is completed once the required purity is achieved.
